# EUROPÄISCHE PATENTSCHRIFT

(11) **EP 1 235 823 B1**
(45) Veröffentlichungstag und Bekanntmachung des Hinweises auf die Patenterteilung: **16.07.2003**
(21) Anmeldenummer: 01956558.9
(22) Anmeldetag: 24.07.2001
(51) Int. Cl.: C07D 311/78, C07D 493/06, C07D 498/06, C07D 311/94

(54) **H-ANNELLIERTE BENZO [F]CHROMENE**
H-ANNELLATED BENZO [F]CHROMENES
BENZO [F]CHROMENES H ANNELES

(30) Priorität: 14.09.2000 DE 10045848
(43) Veröffentlichungstag der Anmeldung: 04.09.2002
(73) Patentinhaber: Optische Werke G. Rodenstock, 80469 München (DE)
(72) Erfinder: MANN, Claudia, 80634 München (DE); MELZIG, Manfred, 82234 Wessling (DE); WEIGAND, Udo, 80638 München (DE)
(74) Vertreter: Müller-Boré & Partner Patentanwälte
(86) Internationale Anmeldenummer: EP0108557
(87) Internationale Veröffentlichungsnummer: WO02022594

(56) Entgegenhaltungen:
- WO-A-99/23071
- US-A- 5 869 658

## Beschreibung

Die vorliegende Erfindung betrifft spezifische photochrome h-annellierte Benzo[f]chromen-Derivate sowie deren Verwendung in Kunststoffen aller Art, insbesondere für ophthalmische Zwecke. Insbesondere betrifft die vorliegende Erfindung von Benzo[f]chromenen abgeleitete photochrome Verbindungen, die in der offenen Form besonders langwellige Absorptionsmaxima aufweisen, wodurch bei Anwendung in phototropen Gläsern violette bis blaue Farbtöne erreicht werden können.

Es sind verschiedene Farbstoffklassen bekannt, die bei Bestrahlung mit Licht bestimmter Wellenlängen, insbesondere Sonnenstrahlen, reversibel ihre Farbe wechseln. Dies rührt daher, daß diese Farbstoffmoleküle durch Energiezufuhr in Form von Licht in einen angeregten farbigen Zustand übergehen, den sie bei Unterbrechung der Energiezufuhr wieder verlassen, wodurch sie in ihren farblosen oder zumindest kaum gefärbten Normalzustand zurückkehren. Zu diesen photochromen Farbstoffen gehören beispielsweise die Naphthopyrane, die im Stand der Technik mit verschiedenen Substituenten bereits beschrieben wurden.

Pyrane, speziell Naphthopyrane und von diesen abgeleitete größere Ringsysteme, sind photochrome Verbindungen, die bis heute Gegenstand intensiver Untersuchungen sind. Obwohl bereits im Jahr 1966 erstmals zum Patent angemeldet (US 3,567,605), konnten erst in den 90er Jahren Verbindungen entwickelt werden, die für den Einsatz in Brillengläsern geeignet erschienen.

Die im Stand der Technik bekannten Farbstoffe weisen dabei zum einen häufig eine nicht ausreichend langwellige Absorption im angeregten wie im nicht angeregten Zustand auf. Dies führt auch bei Kombinationen mit weiteren photochromen Farbstoffen zu Problemen. Zum anderen liegt häufig auch eine zu hohe Temperaturempfindlichkeit bezüglich der Eindunkelung vor, wobei gleichzeitig eine zu langsame Aufhellung eintritt. Darüberhinaus besitzen die beschriebenen Farbstoffe eine ungenügende Lebensdauer. Infolgedessen zeigen derartige Sonnenschutzgläser eine unzureichende Haltbarkeit. Letzteres macht sich in schnell nachlassender Leistung und/oder starker Vergilbung bemerkbar.

Von 2-Naphtholen abgeleitete 3H-Naphthopyrane und ihre davon durch Annellierung abgeleiteten höheren analogen Derivate sind eine Gruppe von photochromen Farbstoffen, deren längstwelliges Absorptionsmaximum der angeregten Form vorwiegend im Spektralbereich von 420 nm bis 500 nm liegt und somit einen gelben, orangen oder roten Farbeindruck vermitteln (siehe US-A-5,869,658 sowie US-A-6,022,495). Für neutraleindunkelnde phototrope Gläser werden jedoch leistungsstarke violette bis blaue photochrome Farbstoffe benötigt. Violette bis blaue photochrome Farbstoffe, die derzeit im Stand der Technik verfügbar sind, stammen aus der Klasse der Spiroxazine, Fulgide oder 2H-Naphtho[1,2-b]pyrane. Spiroxazin-Farbstoffe sind jedoch in der Regel hinsichtlich der Hochtemperaturleistung nachteilig, während Fulgid-Farbstoffe in der Lebensdauer und 2H-Naphtho[1,2-b]pyrane in der Aufhellgeschwindigkeit keine für die Anwendung in Sonnenschutzgläser zufriedenstellenden Eigenschaften aufweisen.

Somit liegt der vorliegenden Erfindung die Aufgabe zugrunde, neue photochrome Farbstoffe bereitzustellen, die verbesserte Eigenschaften gegenüber den im Stand der Technik beschriebenen Verbindungen besitzen sollen. Die photochromen Verbindungen sollen sich gegenüber vergleichbaren Verbindungen aus dem Stand der Technik insbesondere durch eine im angeregten Zustand längerwellige Absorption bei gleichzeitig guten Kinetik- und Lebensdauereigenschaften, d.h. schneller Aufhellungsgeschwindigkeit sowie gutem Verhalten im Lebensdauertest, auszeichnen.

Diese Aufgabe wird durch die in den Ansprüchen gekennzeichneten Gegenstände gelöst.

Insbesondere werden photochrome h-annellierte Benzo[f]chromene mit der allgemeinen Formel (I) bereitgestellt: 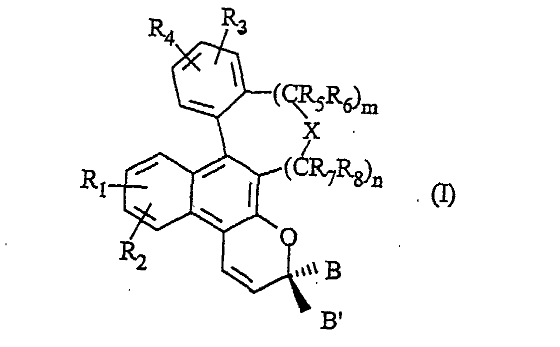 worin
n und m unabhängig voneinander 0, 1 oder 2 bedeuten,
die Reste R₁, R₂, R₃ und R₄ jeweils unabhängig voneinander einen Substituenten darstellen, ausgewählt aus
der Gruppe α, bestehend aus einem Wasserstoffatom, einem (C₁-C₆)-Alkylrest, einem (C₃-C₇)-Cycloalkylrest, der ein oder mehrere Heteroatome aufweisen kann, einem (C₁-C₆)-Alkoxyrest, einer Hydroxygruppe, einer Trifluormethylgruppe, Brom, Chlor und Fluor;
der Gruppe β, bestehend aus einem un-, mono- oder disubstituiertem Phenyl-, Phenoxy-, Benzyl-, Benzyloxy-, Naphthyl- oder Naphthoxyrest, wobei die Substituenten aus der Gruppe α und Phenyl ausgewählt sein können;
der Gruppe χ, worin die Reste R₁ und R₂ bzw. R₃ und R₄ jeweils eine an den aromatischen Ring gebundene -A-(CH₂)ₖ-B- Gruppe mit k = 1 oder 2 bilden, wobei A und B unabhängig voneinander aus Sauerstoff, Schwefel, CH₂; C(CH₃)₂ oder C(C₆H₅)₂ ausgewählt sind, und wobei an diese -A-(CH₂)ₖ-B- Gruppe wiederum ein Benzoring annelliert sein kann;
der Gruppe δ, bestehend aus einer un-, mono- oder disubstituierten Aminogruppe, wobei die Aminsubstituenten aus einem (C₁-C₆)-Alkylrest, einem (C₃-C₇)-Cycloalkylrest, einem unsubstituierten oder mit einem oder mehreren Substituenten aus der Gruppe α substituierten Phenyl- oder Benzylrest ausgewählt sein können, einer N-Morpholingruppe, einer N-Thiomorpholingruppe, einer N-Piperidingruppe, einer N-Azacycloheptangruppe, einer N-Piperazingruppe, einer N-(N'-(C₁-C₆-Alkyl)piperazingruppe, einer N-Pyrrolidingruppe, einer N-Imidazolidingruppe, einer N-Pyrazolidingruppe, einer N-Aziridingruppe, einer N-Azetidingruppe, einer N-Indolingruppe, einer N-Carbazolgruppe, einer N-Phenothiazingruppe, einer N-Phenazingruppe, einer N-Phenoxazingruppe, einer N-Tetrahydrochinolingruppe und einer N-Tetrahydroisochinolingruppe,
der Gruppe ε, worin die Reste R₁ und R₂, d.h. R₁/R₂, bzw. R₃ und R₄, d.h. R₃/R₄, jeweils miteinander eine an den Benzoring gebundene -D-(CH₂)ₖ-E- Gruppe mit k = 1 oder 2 bilden, wobei unabhängig voneinander D aus N(CH₃) oder N(C₆H₅) und
E aus Sauerstoff, Schwefel, CH₂, C(CH₃)₂, C(C₆H₅)₂, N(CH₃) oder N(C₆H₅) ausgewählt sind, und wobei an diese -D-(CH₂)ₖ-E- Gruppe wiederum ein Benzoring annelliert sein kann, oder
der Gruppe φ, worin die Reste R₁ und R₂ bzw. R₃ und R₄ jeweils zusammen mit dem Benzolring, an den sie gebunden sind, eine Julolidinyl-Einheit bilden,
mit der Maßgabe, daß innerhalb der Reste R₁, R₂, R₃ und R₄ bzw. innerhalb R₁/R₂ und R₃/R₄ mindestens einer aus den vorstehenden Gruppen δ, ε und φ ausgewählt ist, d.h. mindestens einer der Reste R₁, R₂, R₃ und R₄ bzw. R₁/R₂ und R₃/R₄ ist ein elektronenliefernder Substituent aus der Klasse der Stickstoffdonor-Substituenten;
die Reste R₅, R₆, R₇ und R₈ jeweils unabhängig voneinander aus der Gruppe α und Phenyl ausgewählt sind oder die Reste R₅ und R₆ zusammen mit dem Rest R₃ des direkt benachbarten Benzoringes einen daran annellierten, un-, monooder disubstituierten Benzo- oder Pyridoring bilden, dessen Substituenten aus der Gruppe α und Phenyl ausgewählt sein können, oder, wenn m bzw. n 2 bedeuten, die direkt benachbarten Reste R₅ und R₆ zweier benachbarter CR₅R₆-Einheiten bzw. die direkt benachbarten Reste R₇ und R₈ zweier benachbarter CR₇R₈-Einheiten miteinander einen annellierten, un-, mono- oder disubstituierten Benzooder Pyridoring bilden, dessen Substituenten aus der Gruppe α und Phenyl ausgewählt sein können, oder die Reste R₅ und R₆ und/oder die Reste R₇ und R₈ zusammen einen (C₃-C₇)-Cycloalkylrest darstellen, der ein oder mehrere Heteroatome, wie beispielsweise Sauerstoff, Stickstoff oder Schwefel, aufweisen kann, wobei an diesen Cycloalkylrest ein Benzoring annelliert sein kann;
X aus O, S, CR₉R₁₀ oder NR₁₁ ausgewählt ist, wobei der Rest R₁₁ Wasserstoff, Cyano, (C₁-C₆)-Alkyl, (C₃-C₇)-Cycloalkyl oder Phenyl sein kann, und die Reste R₉ und R₁₀ unabhängig voneinander aus der Gruppe α und Phenyl ausgewählt sind oder die Reste R₉ und R₁₀ zusammen einen (C₃-C₇)-Cycloalkylrest darstellen, der ein oder mehrere Heteroatome aufweisen kann, oder, wenn X CR₉R₁₀ ist, die Reste R₉ und R₁₀ zusammen mit den Resten R₅ und R₆ bzw. R₇ und R₈ einer direkt benachbarten CR₅R₆-Einheit bzw. CR₇R₈-Einheit auch für einen an die X-C(R₅R₆) bzw. X-C(R₇R₈)-Bindung annellierten, un-, mono- oder disubstituierten Benzo- oder Pyridoring stehen können, dessen Substituenten aus der Gruppe α und Phenyl ausgewählt sein können;
B und B' unabhängig voneinander aus einer der folgenden Gruppen a), b), c) oder d) ausgewählt sind, wobei sie
a) mono-, di- und trisubstituierte Arylreste sind, wobei der Arylrest Phenyl oder Naphthyl ist;
b) unsubstituierte, mono- und disubstituierte Heteroarylreste sind, wobei der Heteroarylrest Pyridyl, Furanyl, Benzofuran-2-yl, Benzofuran-3-yl, Thienyl, Benzothien-2-yl, Benzothien-3-yl oder Julolidinyl ist;
   wobei die Substituenten der Aryl- oder Heteroarylreste in a) und b) solche, ausgewählt aus den Gruppen α, β oder δ, sind oder zwei direkt benachbarte Substituenten miteinander eine an den Benzoring gebundene -D-(CH₂)ₖ-E-Gruppe, wie vorstehend definiert, bilden können, d.h. k steht für 1 oder 2 und unabhängig voneinander stehen D für -N(CH₃) oder -N(C₆H₅) und E für Sauerstoff, Schwefel, CH₂, C(CH₃)₂, C(C₆H₅)₂, -N(CH₃) oder -N(C₆H₅), und an diese -D-(CH₂)ₖ-E- Gruppe kann wiederum ein Benzoring annelliert sein;
c) Struktureinheiten mit den folgenden Formeln (V) und (W) sind: 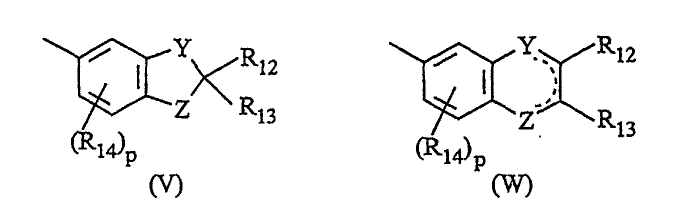 worin
   Y und Z unabhängig voneinander O, S, CH, CH₂ oder NR₁₁ sind, wobei der Rest R₁₁ wie vorstehend definiert ist, die Reste R₁₂ und R₁₃ unabhängig voneinander Wasserstoff und/oder einen (C₁-C₆)-Alkylrest darstellen und der Rest R₁₄ ein Substituent aus der Gruppe α ist, wobei p 1, 2, oder 3 ist, mit der Maßgabe, daß, wenn Y in der Formel (V) NR₁₁ ist, Z Kohlenstoff ist, oder
d) B und B' miteinander einen un-, mono- oder disubstituierten Fluoren-9-ylidenrest oder einen gesättigten Kohlenwasserstoffrest bilden, der C₃-C₁₂ spiro-monozyklisch, C₇-C₁₂ spiro-bizyklisch und/oder C₇-C₁₂ spiro-trizyklisch ist, wobei die Fluorensubstituenten aus der Gruppe α ausgewählt sind.

Gemäß der vorliegenden Erfindung werden durch h-Annellierung von Benzo[f]chromen-Systemen Verbindungen bereitgestellt, deren photochrome Eigenschaften gegenüber den im Stand der Technik bekannten Verbindungen Vorzüge aufweisen. Insbesondere weisen die erfindungsgemäßen Verbindungen besonders langwellige Absorptionsmaxima in der offenen (farbigen) Form auf, so daß erstmals violette bis blaue Farbtöne für Benzo[f]chromen-Systeme (Nomenklatur gemäß IUPAC; andere Bezeichnung: 3H-Naphtho[2,1-b]pyrane) erhalten werden können. Gleichzeitig zeigen die erfindungsgemäßen photochromen h-annellierten Benzo[f]chromen-Derivate gegenüber entsprechenden Verbindungen aus dem Stand der Technik vergleichbar gute Kinetik- und Lebensdauereigenschaften, d.h. schnelle Aufhellungsgeschwindigkeit sowie gutes Verhalten im Lebensdauertest.

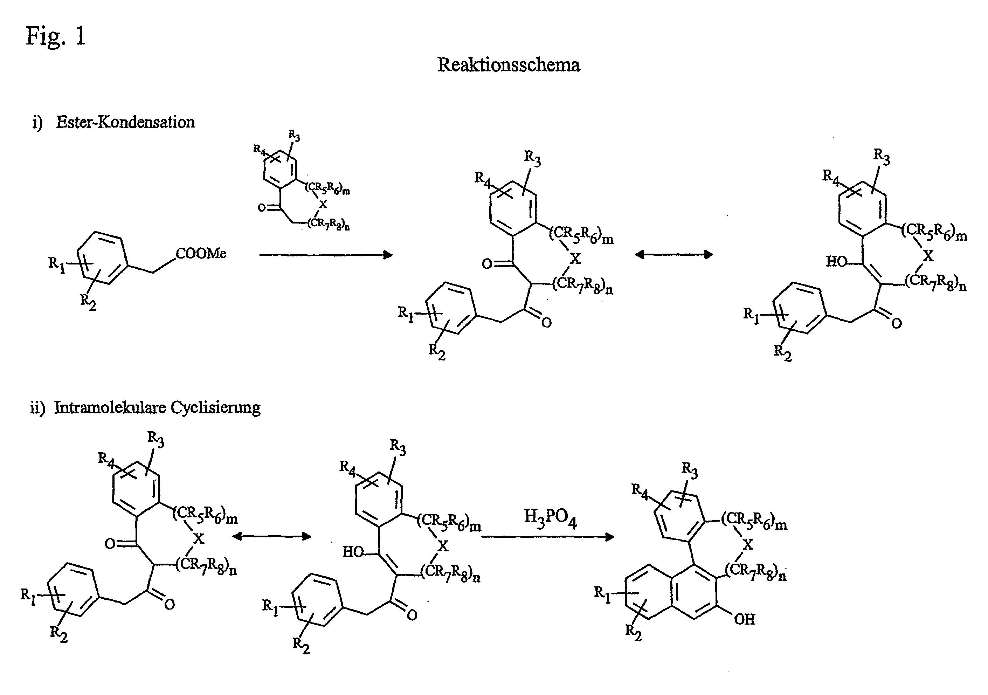

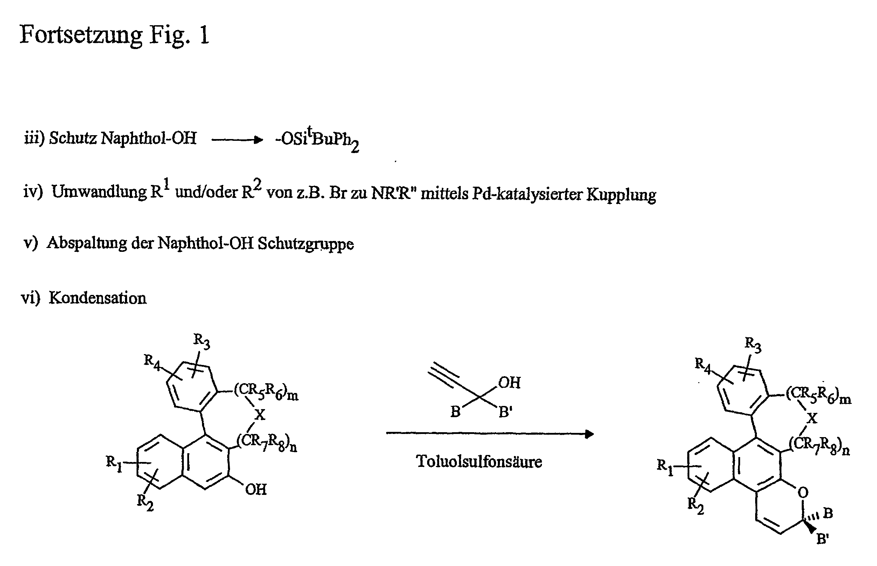

Fig. 1 zeigt einen Syntheseweg zur Herstellung beispielhafter erfindungsgemäßer photochromer Verbindungen.

Der in h-Position des Benzo[f]chromen-Systems annellierte Cyclus bzw. Heterocyclus ist vorzugsweise ein Fünfring (n = m = 0), Sechsring (n = 1, m = 0 bzw. m = 1, n = 0) oder Siebenring (mit vorzugsweise n = m = 1).

Bevorzugte photochrome h-annellierte Benzo[f]chromen-Derivate gemäß der vorliegenden Erfindung weisen die nachfolgenden allgemeinen Formeln (II) bzw. (III) auf: 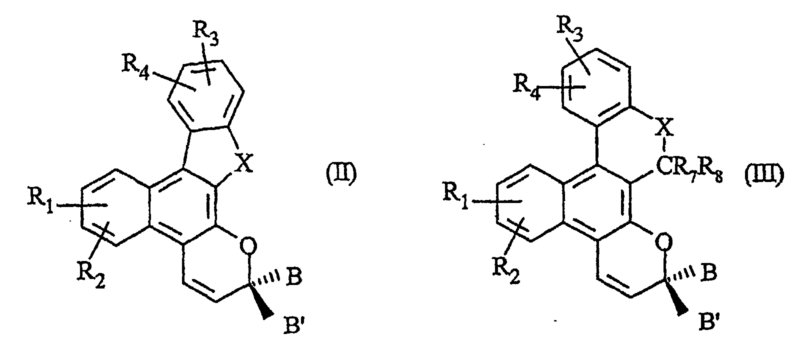 worin B, B', R₁, R₂, R₃ ,R₄, R₇ ,R₈ und X wie vorgenannt definiert sind.

Vorzugsweise werden die Reste R₁, R₂, R₃ und R₄ gemäß der vorstehenden Formeln (I), (II) bzw. (III) aus den Gruppen α, β und δ ausgewählt, mit der Maßgabe, daß innerhalb der Reste R₁, R₂, R₃ und R₄ mindestens einer aus der vorstehenden Gruppe δ ausgewählt ist. Besonders bevorzugt ist mindestens einer der Reste R₁ und R₂ aus der Gruppe δ ausgewählt. Noch mehr bevorzugt ist der Rest R¹ aus der Gruppe δ ausgewählt, wobei der Rest R₁ in 7-Position des h-annellierten 2H-Benzo[f]chromen-Systems gebunden ist. Innerhalb der Gruppe δ sind eine un-, mono- oder disubstituierten Aminogruppe, wobei die Aminsubstituenten aus einem (C₁-C₆)-Alkylrest, einem (C₃-C₇)-Cycloalkylrest, einem unsubstituierten oder mit einem oder mehreren Substituenten aus der Gruppe α substituierten Phenyl- oder Benzylrest ausgewählt sein können, eine N-Morpholingruppe, eine N-Piperidingruppe, eine N-Azacycloheptangruppe, eine N-Piperazingruppe, eine N-(N'-(C₁-C₆-Alkyl)piperazingruppe, eine N-Pyrrolidingruppe, eine N-lmidazolidingruppe und eine N-Pyrazolidingruppe besonders bevorzugt.

Die Reste R₅, R₆, R₇ und R₈ werden jeweils unabhängig voneinander vorzugsweise aus der Gruppe α ausgewählt. X kann insbesondere O, CR₉R₁₀ oder NR₁₁ sein, wobei die Reste R₉, R₁₀ und R₁₁ wie vorgenannt definiert sind. Wenn X für CR₉R₁₀ steht, können die Reste R₉ und R₁₀ miteinander insbesondere für einen (C₃-C₇)-Cycloalkylrest stehen, der ein oder mehrere Heteroatome aufweisen kann.

In einer besonders bevorzugten Ausführungsform sind in den vorstehend aufgeführten Formeln (I) bzw. (II) und (III) B und B' unabhängig voneinander mono-, di- und trisubstituierte Acylreste, wobei der Arylrest jeweils ein Phenylrest oder ein Naphthylrest ist.

Wenn die Reste R₁ und R₂ bzw. R₃ und R₄ jeweils zusammen mit dem Benzolring, an den sie gebunden sind, d.h. mit dem in der f-Position an der 2H-Chromen-Einheit annellierten Benzoring, eine Julolidinyl-Einheit bilden, ergibt sich folgende Struktureinheit:

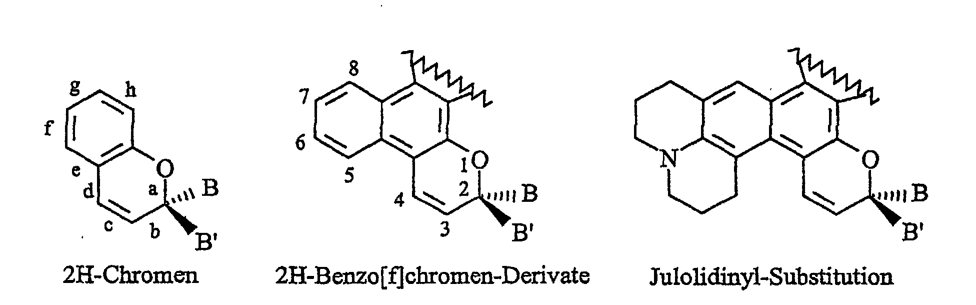

Als -A-(CH₂)ₖ-B- Einheit kann insbesondere -O-(CH₂)₂-O- angeführt werden, wobei gegebenfalls an die Ethylengruppe davon ein Benzocyclus annelliert ist. Als -D-(CH₂)ₖ-E- Einheit können insbesondere die folgenden angeführt werden:
-NMe-(CH₂)₂-NMe-, -NPh-(CH₂)₂-NPh-, -NMe-(CH₂)₂-O-, -NPh-(CH₂)₂-NMe- und
-NMe-(CH₂)₂-C(CH₃)₂-.

Die -A-(CH₂)ₖ-B- Einheit bzw. -D-(CH₂)ₖ-E- Einheit ist dabei über A und B bzw. D und E in ortho-Stellung zueinander an den jeweiligen Benzoring gebunden.

Besonders bevorzugte photochrome h-annellierte Benzo[f]chromen-Derivate gemäß der vorliegenden Erfindung sind:
(1) 7-Dimethylamino-6-methoxy-2-(4-(N-morpholinyl)phenyl)-2-phenyl-2H,13Hindeno[1,2-h]benzo[f]chromen,
(2) 7-Dimethylamino-6-methoxy-2-(4-(N-morpholinyl)phenyl)-2,13,13-triphenyl-2H,13H-indeno[1,2-h]benzo[f]chromen,
(3) 7-Dimethylamino-6-methoxy-2-(4-(N-morpholinyl)phenyl)-2-phenyl-2H-13,14-dihydro-naphtho[1,2-h]benzo[f]chromen,
(4) 7-Dimethylamino-6-methoxy-2-(4-(N-morpholinyl)phenyl)-2-phenyl-2Hbenzofuro[3,2-h]benzo[f]chromen,
(5) 2-(4-Dimethylaminophenyl)-6-methoxy-7-(N-piperidinyl)-2-phenyl-2H,13Hindeno[1 ,2-h]benzo[f]chromen,
(6) 2-(4-Dimethylaminophenyl)-6-methoxy-7-(N-piperidinyl)-2,13,13-triphenyl-2H,13H-indeno[1,2-h]benzo[f]chromen,
(7) 2-(4-Dimethylaminophenyl)-6-methoxy-7-(N-piperidinyl)-2-phenyl-2H-13,14-dihydro-naphtho[1,2-h]benzo[f]chromen,
(8) 2-(4-Dimethylaminophenyl)-6-methoxy-7-(N-piperidinyl)-2-phenyl-2Hbenzofuro[3,2-h]benzo[flchromen,
(9) 2-(4-Dimethylaminophenyl)-6-methoxy-7-(N-morpholinyl)-2-phenyl-2H,13Hindeno[1,2-h]benzo[f]chromen,
(10) 2-(4-Dimethylaminophenyl)-6-methoxy-7-(N-morpholinyl)-2,13,13-triphenyl-2H,13H-indeno[1,2-h]benzo[f]chromen,
(11) 2-(4-Dimethylaminophenyl)-6-methoxy-7-(N-morpholinyl)-2-phenyl-2H-13,14-dihydro-naphtho[1,2-h]benzo[f]chromen,
(12) 2-(4-Dimethylaminophenyl)-6-methoxy-7-(N-morpholinyl)-2-phenyl-2Hbenzofuro[3,2-h]benzo[f]chromen,
(13) 7-Methoxy-6-(N-morpholinyl)-2-(4-(N-morpholinyl)phenyl)-2-phenyl-2H,13Hindeno[1,2-h]benzo[f]chromen,
(14) 7-Methoxy-6-(N-morpholinyl)-2-(4-(N-morpholinyl)phenyl)-2,13,13-triphenyl-2H,13H-indeno[1,2-h]benzo[f]chromen,
(15) 7-Methoxy-6-(N-morpholinyl)-2-(4-(N-morpholinyl)phenyl)-2-phenyl-2H-13,14-dihydro-naphtho[1,2-h]benzo[flchromen,
(16) 7-Methoxy-6-(N-morpholinyl)-2-(4-(N-morpholinyl)phenyl)-2-phenyl-2H-benzofuro[3,2-h]benzo[f]chromen,
(17) 2-(4-Dimethylaminophenyl)-9-methyl-2-phenyl-2H,9H,15H-7,8-dihydroindeno[1,2-h]benz[1,4]oxazino[6,7-f]chromen,
(18) 2-(4-Dimethylaminophenyl)-9-methyl-2,15,15-triphenyl-2H,9H,15H-7,8-dihydroindeno[1,2-h]benz[1,4]oxazino[6,7-f]chromen,
(19) 2-(4-Dimethylaminophenyl)-9-methyl-2-phenyl-2H,9H-7,8,15,16-tetrahydronaphtho[1,2-h]benz[1,4]oxazino[6,7-f]chromen und
(20) 2-(4-Dimethylaminophenyl)-9-methyl-2-phenyl-2H,9H-benzofuro[3,2-h]benz[1,4]-oxazino[6,7-f]chromen.

Die längstwelligen Absorptionsmaxima der offenen Form der vorgenannten beispielhaften photochromen h-annellierten Benzo[f]chromen-Derivate gemäß der vorliegenden Erfindung sind in der nachstehenden Tabelle exemplarisch aufgeführt.

| Verbindungen | längstwelliges Absorptionsmaximum der offenen (farbigen) Form |
|---|---|
| (1) - (4) | 570 - 575 nm |
| (5) - (8) | 585 - 595 nm |
| (9) - (12) | 565 - 575 nm |
| (13) - (16) | 545 - 555 nm |
| (17) - (20) | 590 - 600 nm |

Die erfindungsgemäßen Verbindungen können in Kunststoffmaterialien bzw. Kunststoffgegenständen jeglicher Art und Form für eine Vielzahl von Einsatzzwecken, für die photochromes Verhalten von Bedeutung ist, verwendet werden. Dabei können ein Farbstoff gemäß der vorliegenden Erfindung oder ein Gemisch solcher Farbstoffe eingesetzt werden. Beispielsweise können die erfindungsgemäßen photochromen Benzo[f]chromen-Farbstoffe in Linsen, insbesondere ophthalmischen Linsen, Gläsern für Brillen aller Art, wie beispielsweise Skibrillen, Sonnenbrillen, Motorradbrillen, Visieren von Schutzhelmen, und dergleichen eingesetzt werden. Ferner können die erfindungsgemäßen photochromen Benzo[f]chromen-Farbstoffe beispielsweise auch als Sonnenschutz in Fahrzeugen und Wohnräumen in Form von Fenstern, Schutzblenden, Abdeckungen, Dächern oder dergleichen verwendet werden.

Zur Herstellung von solchen photochromen Gegenständen können die erfindungsgemäßen photochromen Benzo[f]chromen-Farbstoffe durch verschiedene, im Stand der Technik beschriebene Verfahren, wie bereits in WO 99/15518 angegeben, auf ein Polymermaterial, wie ein organisches Kunststoffmaterial, aufgebracht oder darin eingebettet werden.

Es werden dabei sogenannte Massefärbungs- und Oberflächenfärbungsverfahren unterschieden. Ein Massefärbungsverfahren umfaßt beispielsweise das Auflösen oder Dispergieren der photochromen Verbindung oder Verbindungen gemäß der vorliegenden Erfindung in einem Kunststoffmaterial, z.B. durch die Zugabe der photochromen Verbindung(en) zu einem monomeren Material, bevor die Polymerisation erfolgt. Eine weitere Möglichkeit zur Herstellung eines photochromen Gegenstands ist die Durchdringung des oder der Kunststoffmaterialien mit der (den) photochromen Verbindung(en) durch Eintauchen des Kunststoffmaterials in eine heiße Lösung des oder der photochromen Farbstoffe gemäß der vorliegenden Erfindung oder beispielsweise auch ein Thermotransferverfahren. Die photochrome(n) Verbindung(en) kann bzw. können beispielsweise auch in Form einer separaten Schicht zwischen aneinandergrenzenden Schichten des Kunststoffmaterials, z.B. als Teil eines polymeren Films, vorgesehen werden. Ferner ist auch ein Aufbringen der photochromen Verbindung(en) als Teil einer auf der Oberfläche des Kunststoffmaterials befindlichen Beschichtung möglich. Der Ausdruck "Durchdringung" soll dabei die Migration der photochromen Verbindung(en) in das Kunststoffmaterial, z.B. durch den lösungsmittelunterstützten Transfer der photochromen Verbindung(en) in eine Polymermatrix, Dampfphasentransfer oder andere derartige Oberflächendiffusionsvorgänge, bedeuten. Vorteilhafterweise können solche photochromen Gegenstände, wie z.B. Brillengläser, nicht nur mittels der üblichen Massefärbung, sondern in gleicher Weise auch mittels Oberflächenfärbung hergestellt werden, wobei bei der letzteren Variante eine überraschend geringere Migrationsneigung erzielt werden kann. Dies ist vor allem bei nachfolgenden Veredelungsschritten von Vorteil, da - z.B. bei einer Antireflexbeschichtung durch die geringere Rückdiffusion im Vakuum - Schicht-ablösungen und ähnliche Defekte drastisch verringert werden.

Insgesamt können auf Basis der erfindungsgemäßen photochromen h-annellierten Benzo[f]chromen-Derivate beliebig kompatible (in chemischer Hinsicht und farblicher Art und Weise verträgliche) Färbungen, d.h. Farbstoffe, auf das Kunststoffmaterial aufgebracht oder in es eingebettet werden, um sowohl ästhetischen Gesichtspunkten als auch medizinischen oder modischen Aspekten zu genügen. Der oder die spezifisch ausgewählte(n) Farbstoff(e) kann bzw. können demzufolge, abhängig von den beabsichtigten Wirkungen sowie Anforderungen, variieren.

Die erfindungsgemäßen photochromen h-annellierten Benzo[f]chromen-Derivate mit der allgemeinen Formel (I) bzw. (II) und (III) lassen sich beispielsweise nach dem in Figur 1 gezeigten Reaktionsschema herstellen.

Ausgehend von geeignet substituierten Phenylessigsäureestem wird eine Ester-Kondensation mit cyclischen aromatisch-aliphatischen Ketonen unter Verwendung von Kalium-methylat als Base durchgeführt; vgl. Schritt i) in Figur 1. Die in Schritt i) erhaltenen substituierten cyclischen aromatisch-aliphatischen 2-Phenylacetyl-Ketone werden anschließend gemäß Schritt ii) in einer intramolekularen Cyclisierung mittels Phosphorsäure zu substituierten annellierten 2-Naphthol-Derivaten umgesetzt. Nach Schutz der Naphthol-Hydroxygruppe als tert.-Butyldiphenysilylether (Schritt iii)) wird die für die langwellige Absorption benötigte (substituierte) Amino-Gruppe am Naphthalin-System mittels Palladiumkatalysierter Aminierung eingeführt (Schritt iv)). Die hierfür in Schritt iv) erforderlichen Ausgangsverbindungen tragen als Reste R₁ oder R₂ entweder ein Bromatom oder eine Triflatgruppe, welche entweder von Anfang an vorhanden sein können, d.h. in dem in Schritt i) als Ausgangsmaterial entsprechend verwendeten Phenylessigsäureester, oder in herkömmlicher Weise unmittelbar vor der Aminierung eingeführt werden können. Nach Abspaltung der Silylether-Schutzgruppe in Schritt v) werden die derart erhaltenen substituierten annellierten 2-Naphthol-Derivate mit geeignet substituierten 2-Propin-1-ol-Derivaten gemäß Schritt vi) zu den erfindungsgemäßen Verbindungen umgesetzt.

## Patentansprüche

1. Photochrome h-annellierte Benzo[f]chromene mit der allgemeinen Formel (I): 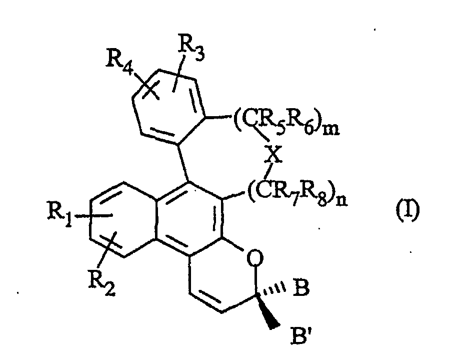 worin
n und m unabhängig voneinander 0, 1 oder 2 bedeuten,
die Reste R₁, R₂, R₃ und R₄ jeweils unabhängig voneinander einen Substituenten darstellen, ausgewählt aus
der Gruppe α, bestehend aus einem Wasserstoffatom, einem (C₁-C₆)-Alkylrest, einem (C₃-C₇)-Cycloalkylrest, der ein oder mehrere Heteroatome aufweisen kann, einem (C₁-C₆)-Alkoxyrest, einer Hydroxygruppe, einer Trifluormethylgruppe, Brom, Chlor und Fluor;
der Gruppe β, bestehend aus einem un-, mono- oder disubstituiertem Phenyl-, Phenoxy-, Benzyl-, Benzyloxy-, Naphthyl- oder Naphthoxyrest, wobei die Substituenten aus der Gruppe α und Phenyl ausgewählt sein können;
der Gruppe χ, worin die Reste R₁ und R₂ bzw. R₃ und R₄ jeweils eine an den aromatischen Ring gebundene -A-(CH₂)ₖ-B- Gruppe mit k = 1 oder 2 bilden, wobei A und B unabhängig voneinander aus Sauerstoff, Schwefel, CH₂, C(CH₃)₂ oder C(C₆H₅)₂ ausgewählt sind, und wobei an diese -A-(CH₂)ₖ-B- Gruppe wiederum ein Benzoring annelliert sein kann;
der Gruppe δ, bestehend aus einer un-, mono- oder disubstituierten Aminogruppe, wobei die Aminsubstituenten aus einem (C₁-C₆)-Alkylrest, einem (C₃-C₇)-Cycloalkylrest, einem unsubstituierten oder mit einem oder mehreren Substituenten aus der Gruppe α substituierten Phenyl- oder Benzylrest ausgewählt sein können, einer N-Morpholingruppe, einer N-Thiomorpholingruppe, einer N-Piperidingruppe, einer N-Azacycloheptangruppe, einer N-Piperazingruppe, einer N-(N'-(C₁-C₆-Alkyl)piperazingruppe, einer N-Pyrrolidingruppe, einer N-Imidazolidingruppe, einer N-Pyrazolidingruppe, einer N-Aziridingruppe, einer N-Azetidingruppe, einer N-Indolingruppe, einer N-Carbazolgruppe, einer N-Phenothiazingruppe, einer N-Phenazingruppe, einer N-Phenoxazingruppe, einer N-Tetrahydrochinolingruppe und einer N-Tetrahydroisochinolingruppe,
der Gruppe ε, worin die Reste R₁ und R₂ bzw. R₃ und R₄ jeweils miteinander eine an den Benzoring gebundene -D-(CH₂)ₖ-E- Gruppe mit k = 1 oder 2 bilden, wobei unabhängig voneinander D aus N(CH₃) oder N(C₆H₅) und E aus Sauerstoff, Schwefel, CH₂, C(CH₃)₂, C(C₆H₅)₂, N(CH₃) oder N(C₆H₅) ausgewählt sind, und wobei an diese -D-(CH₂)ₖ-E- Gruppe wiederum ein Benzoring annelliert sein kann, oder
der Gruppe φ, worin die Reste R₁ und R₂ bzw. R₃ und R₄ jeweils zusammen mit dem Benzolring, an den sie gebunden sind, eine Julolidinyl-Einheit bilden,
mit der Maßgabe, daß innerhalb der Reste R₁, R₂, R₃ und R₄ bzw. innerhalb R₁/R₂ und R₃/R₄ mindestens einer aus den vorstehenden Gruppen δ, ε und φ ausgewählt ist,
die Reste R₅, R₆, R₇ und R₈ jeweils unabhängig voneinander aus der Gruppe α und Phenyl ausgewählt sind oder die Reste R₅ und R₆ zusammen mit dem Rest R₃ des direkt benachbarten Benzoringes einen daran annellierten, un-, monooder disubstituierten Benzo- oder Pyridoring bilden, dessen Substituenten aus der Gruppe α und Phenyl ausgewählt sein können, oder, wenn m bzw. n 2 bedeuten, die direkt benachbarten Reste R₅ und R₆ zweier benachbarter CR₅R₆-Einheiten bzw. die direkt benachbarten Reste R₇ und R₈ zweier benachbarter CR₇R₈-Einheiten miteinander einen annellierten, un-, mono- oder disubstituierten Benzooder Pyridoring bilden, dessen Substituenten aus der Gruppe α und Phenyl ausgewählt sein können, oder die Reste R₅ und R₆ und/oder die Reste R₇ und R₈ zusammen einen (C₃-C₇)-Cycloalkylrest darstellen, der ein oder mehrere Heteroatome aufweisen kann, wobei an diesen Cycloalkylrest ein Benzoring annelliert sein kann;
X aus O, S, CR₉R₁₀ oder NR₁₁ ausgewählt ist, wobei der Rest R₁₁ Wasserstoff, Cyano, (C₁-C₆)-Alkyl, (C₃-C₇)-Cycloalkyl oder Phenyl sein kann, und die Reste R₉ und R₁₀ unabhängig voneinander aus der Gruppe α und Phenyl ausgewählt sind oder die Reste R₉ und R₁₀ zusammen einen (C₃-C₇)-Cycloalkylrest darstellen, der ein oder mehrere Heteroatome aufweisen kann, oder, wenn X CR₉R₁₀ ist, die Reste R₉ und R₁₀ zusammen mit den Resten R₅ und R₆ bzw. R₇ und R₈ einer direkt benachbarten CR₅R₆-Gruppe bzw. CR₇R₈-Einheit auch für einen an die X-C(R₅R₆) bzw. X-C(R₇R₈)-Bindung annellierten, un-, mono- oder disubstituierten Benzo- oder Pyridoring stehen können, dessen Substituenten aus der Gruppe α und Phenyl ausgewählt sein können;
B und B' unabhängig voneinander aus einer der folgenden Gruppen a), b), c) oder d) ausgewählt sind, wobei sie
a) mono-, di- und trisubstituierte Arylreste sind, wobei der Arylrest Phenyl oder Naphthyl ist;
b) unsubstituierte, mono- und disubstituierte Heteroarylreste sind, wobei der Heteroarylrest Pyridyl, Furanyl, Benzofuran-2-yl, Benzofuran-3-yl, Thienyl, Benzothien-2-yl, Benzothien-3-yl oder Julolidinyl ist;
wobei die Substituenten der Aryl- oder Heteroarylreste in a) und b) solche, ausgewählt aus den Gruppen α, β oder δ, sind oder zwei direkt benachbarte Substituenten miteinander eine an den Benzoring gebundene -D-(CH₂)ₖ-E-Gruppe, wie vorstehend definiert, bilden können,
c) Struktureinheiten mit den folgenden Formeln (V) und (W) sind: 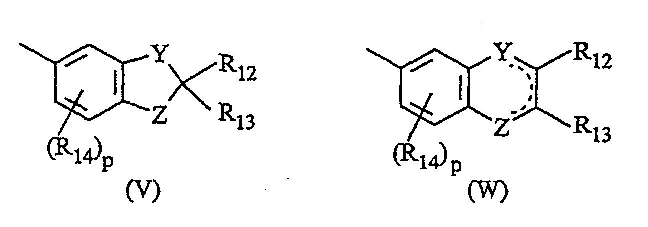 worin
Y und Z unabhängig voneinander O, S, CH, CH₂ oder NR₁₁ sind, wobei der Rest R₁₁ wie vorstehend definiert ist, die Reste R₁₂ und R₁₃ unabhängig voneinander Wasserstoff und/oder einen (C₁-C₆)-Alkylrest darstellen und der Rest R₁₄ ein Substituent aus der Gruppe α ist, wobei p 1, 2, oder 3 ist, mit der Maßgabe, daß, wenn Y in der Formel (V) NR₁₁ ist, Z Kohlenstoff ist, oder
d) B und B' miteinander einen un-, mono- oder disubstituierten Fluoren-9-ylidenrest oder einen gesättigten Kohlenwasserstoffrest bilden, der C₃-C₁₂ spiro-monozyklisch, C₇-C₁₂ spiro-bizyklisch und/oder C₇-C₁₂ spiro-trizyklisch ist, wobei die Fluorensubstituenten aus der Gruppe α ausgewählt sind.

2. Photochrome Benzo[f]chromene nach Anspruch 1, wobei der gemäß Formel (I) in h-Position des Benzo[f]chromen-Systems annellierte Cyclus bzw. Heterocyclus ein Fünfring, Sechsring oder Siebenring ist

3. Photochrome Benzo[f]chromene nach Anspruch 1 oder 2, welche die nachfolgenden allgemeinen Formeln (II) bzw. (III) aufweisen: 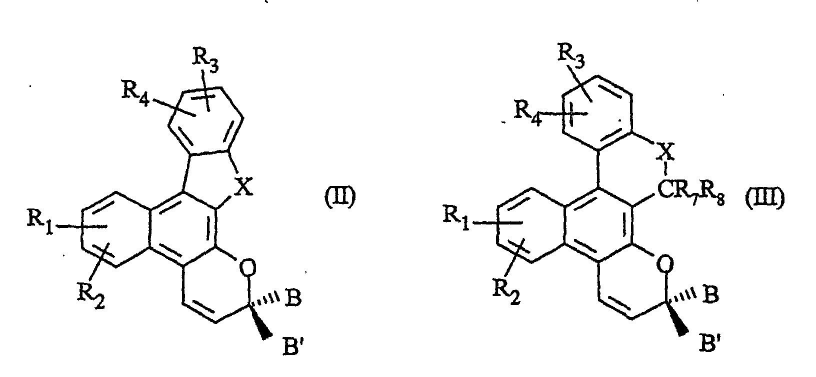 worin B, B', R₁, R₂, R₃ ,R₄, R₇ ,R₈ und X wie vorgenannt definiert sind.

4. Photochrome Benzo[f]chromene nach einem der Ansprüche 1 bis 3, wobei die Reste R₁, R₂, R₃ und R₄ aus den Gruppen α, β und δ ausgewählt sind, mit der Maßgabe, daß innerhalb der Reste R₁, R₂, R₃ und R₄ mindestens einer aus der vorstehenden Gruppe δ ausgewählt ist

5. Photochrome Benzo[f]chromene nach Anspruch 4, wobei mindestens einer der Reste R₁ und R₂ aus der Gruppe δ ausgewählt ist.

6. Photochrome Benzo[f]chromene nach Anspruch 5, wobei der Rest R₁ aus der Gruppe δ ausgewählt und in 7-Position des h-annellierten 2H-Benzo[f]chromen-Systems gebunden ist.

7. Photochrome Benzo[f]chromene nach einem der Ansprüche 1 bis 6, wobei der Substituent aus der Gruppe δ eine un-, mono- oder disubstituierten Aminogruppe, wobei die Aminsubstituenten aus einem (C₁-C₆)-Alkylrest, einem (C₃-C₇)-Cycloalkylrest, einem unsubstituierten oder mit einem oder mehreren Substituenten aus der Gruppe α substituierten Phenyl- oder Benzylrest ausgewählt sein können, eine N-Morpholingruppe, eine N-Piperidingruppe, eine N-Azacycloheptangruppe, eine N-Piperazingruppe, eine N-(N'-(C₁-C₆-Alkyl)piperazingruppe, eine N-Pyrrolidingruppe, eine N-Imidazolidingruppe oder eine N-Pyrazolidingruppe ist.

8. Photochrome Benzo[f]chromene nach einem der Ansprüche 1 bis 7, wobei die Reste R₅, R₆, R₇ und R₈ jeweils unabhängig voneinander aus der Gruppe α ausgewählt sind.

9. Photochrome Benzo[f]chromene nach einem der Ansprüche 1 bis 8, wobei X O, CR₉R₁₀ oder NR₁₁ ist, wobei die Reste R₉, R₁₀ und R₁₁ wie vorgenannt definiert sind.

10. Photochrome Benzo[f]chromene nach einem der vorhergehenden Ansprüche, welche
7-Dimethylamino-6-methoxy-2-(4-(N-morpholinyl)phenyl)-2-phenyl-2H,13Hindeno[1,2-h]benzo[f]chromen,
7-Dimethylamino-6-methoxy-2-(4-(N-morpholinyl)phenyl)-2,13,13-triphenyl-2H,13H-indeno[1,2-h]benzo[f]chromen,
7-Dimethylamino-6-methoxy-2-(4-(N-morpholinyl)phenyl)-2-phenyl-2H-13,14-dihydro-naphtho[1,2-h]benzo[f]chromen,
7-Dimethylamino-6-methoxy-2-(4-(N-morpholinyl)phenyl)-2-phenyl-2Hbenzofuro[3,2-h]benzo[f]chromen,
2-(4-Dimethylaminophenyl)-6-methoxy-7-(N-piperidinyl)-2-phenyl-2H,13Hindeno[1,2-h]benzo[f]chromen,
2-(4-Dimethylaminophenyl)-6-methoxy-7-(N-piperidinyl)-2,13,13-triphenyl-2H,13Hindeno[1,2-h]benzo[f]chromen,
2-(4-Dimethylaminophenyl)-6-methoxy-7-(N-piperidinyl)-2-phenyl-2H-13,14-dihydro-naphtho[1,2-h]benzo[f]chromen,
2-(4-Dimethylaminophenyl)-6-methoxy-7-(N-piperidinyl)-2-phenyl-2Hbenzofuro[3,2-h]benzo[f]chromen,
2-(4-Dimethylaminophenyl)-6-methoxy-7-(N-morpholinyl)-2-phenyl-2H,13Hindeno[1,2-h]benzo[f]chromen,
2-(4-Dimethylaminophenyl)-6-methoxy-7-(N-morpholinyl)-2,13,13-triphenyl-2H,13H-indeno[1,2-h]benzo[f]chromen,
2-(4-Dimethylaminophenyl)-6-methoxy-7-(N-morpholinyl)-2-phenyl-2H-13,14-dihydro-naphtho[1,2-h]benzo[f]chromen,
2-(4-Dimethylaminophenyl)-6-methoxy-7-(N-morpholinyl)-2-phenyl-2Hbenzofuro[3,2-h]benzo[f]chromen,
7-Methoxy-6-(N-morpholinyl)-2-(4-(N-morpholinyl)phenyl)-2-phenyl-2H,13Hindeno[1,2-h]benzo[f]chromen,
7-Methoxy-6-(N-morpholinyl)-2-(4-(N-morpholinyl)phenyl)-2,13,13-triphenyl-2H,13H-indeno[1,2-h]benzo[f]chromen,
7-Methoxy-6-(N-morpholinyl)-2-(4-(N-morpholinyl)phenyl)-2-phenyl-2H-13,14-dihydro-naphtho[1,2-h]benzo[f]chromen,
7-Methoxy-6-(N-morpholinyl)-2-(4-(N-morpholinyl)phenyl)-2-phenyl-2Hbenzofuro[3,2-h]benzo[f]chromen,
2-(4-Dimethylaminophenyl)-9-methyl-2-phenyl-2H,9H,15H-7,8-dihydroindeno[1,2-h]benz[1,4]oxazino[6,7-f]chromen,
2-(4-Dimethylaminophenyl)-9-methyl-2,15,15-triphenyl-2H,9H,15H-7,8-dihydroindeno[1,2-h]benz[1,4]oxazino[6,7-f]chromen,
2-(4-Dimethylaminophenyl)-9-methyl-2-phenyl-2H,9H-7,8,15,16-tetrahydronaphtho[1,2-h]benz[1,4]oxazino[6,7-f]chromen und
2-(4-Dimethylaminophenyl)-9-methyl-2-phenyl-2H,9H-benzofuro[3,2-h]benz[1,4]-oxazino[6,7-f]chromen sind.

11. Verwendung der photochromen Benzo[f]chromene nach einem der Ansprüche 1 bis 10 in Kunststoffmaterialien.

12. Verwendung nach Anspruch 11, wobei das Kunststoffmaterial eine ophthalmische Linse ist.

## Claims

1. Photochromic h-anellated benzo[f]chromenes with the general formula (I): 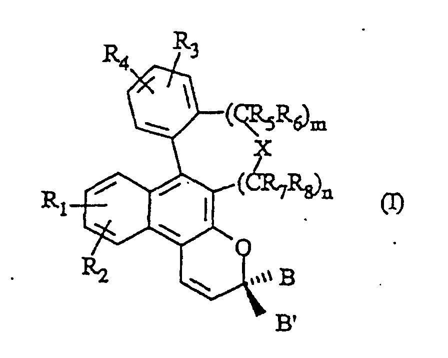 wherein
n and m mean independently of each other 0, 1 or 2,
the radicals R₁, R₂, R₃ and R₄ respectively represent independently of each other a substituent selected from
the group υ̂, comprising a hydrogen atom, a (C₁-C₆)-alkyl radical, a (C₃-C₇)-cycloalkyl radical, which can have one or more heteroatoms, a (C₁-C₆)-alkoxy radical, a hydroxy group, a trifluoromethyl group, bromine, chlorine and fluorine;
the group β, comprising a non-, mono- or disubstituted phenyl-, phenoxy-, benzyl-, benzyloxy-, naphthyl- or naphthoxy radical, the substituents being able to be selected from the group α and phenyl;
the group x, wherein the radicals R₁ and R₂ or R₃ and R₄ respectively form an -A-(CH₂)ₖ-B- group, bonded to the aromatic ring, with k = 1 or 2, A and B being selected independently of each other from oxygen, sulphur, CH₂, C(CH₃)₂ or C(C₆H₅)₂, and a benzo ring being able to be anellated in turn on this -A-(CH₂)ₖ-B- group;
the group δ, comprising a non-, mono- or disubstituted amino group, the amine substituents being able to be selected from a (C₁-C₆)-alkyl radical, a (C₃-C₇)-cycloalkyl radical, a non-substituted phenyl radical or benzyl radical or a phenyl radical or benzyl radical substituted with one or more substituents from the group υ̂, an N-morpholine group, an N-thiomorpholine group, an N-piperidine group, an N-azacycloheptane group, an N-piperazine group, an N-(N'-(C₁-C₆-alkyl)piperazine group, an N-pyrrolidine group, an N-imidazolidine group, an N-pyrazolidine group, an N-aziridine group, an N-azetidine group, an N-indoline group, an N-carbazole group, an N-phenothiazine group, an N-phenazine group, an N-phenoxazine group, an N-tetrahydroquinoline group and an N-tetrahydroisoquinoline group,
the group e, wherein the radicals R₁ and R₂ or R₃ and R₄ respectively form together a -D-(CH₂)ₖ-E- group, bonded to the benzo ring, with k = 1 or 2, D being selected from N(CH₃) or N(C₆H₅) and E from oxygen, sulphur, CH₂, C(CH₃)₂, C(C₆H₅)₂, N(CH₃) or N(C₆H₅), independently of each other, and a benzo ring being able to be anellated in turn on this -D-(CH₂)ₖ-E- group, or
the group φ wherein the radicals R₁ and R₂ or R₃ and R₄ respectively form together with the benzene ring to which they are bonded a julolidinyl unit,
with the proviso that, within the radicals R₁, R₂, R₃ and R₄ or within R₁/R₂ and R₃/R₄, at least one is selected from the above mentioned groups δ, £ and φ,
the radicals R₅, R₆, R₇ and R₈ respectively are selected independently of each other from the group υ̂ and phenyl, or the radicals R₅ and R₆, together with the radical R₃ of the directly adjacent benzo ring, form a non-, mono- or disubstituted benzo ring or pyrido ring anellated thereon, the substituents of which can be selected from the group υ̂ and phenyl or, if m or n mean 2, the directly adjacent radicals R₅ and R₆ of two adjacent CR₅R₆ units or the directly adjacent radicals R₇ and R₈ of two adjacent CR₇R₈ units form together an anellated non-, mono- or disubstituted benzo ring or pyrido ring, the substituents of which can be selected from the group υ̂ and phenyl, or the radicals R₅ and R₆ and/or the radicals R₇ and R₈ represent together a (C₃-C₇)-cycloalkyl radical which can have one or more heteroatoms, a benzo ring being able to be anellated on this cycloalkyl radical;
X is selected from O, S, CR₉R₁₀ or NR₁₁, the radical R₁₁ being able to be hydrogen, cyano, (C₁-C₆)-alkyl, (C₃-C₇)-cycloalkyl or phenyl, and the radicals R₉ and R₁₀ are selected independently of each other from the group υ̂ and phenyl or the radicals R₉ and R₁₀ represent together a (C₃-C₇)-cycloalkyl radical which can have one or more heteroatoms or, if X is CR₉R₁₀, the radicals R₉ and R₁₀ together with the radicals R₅ and R₆ or R₇ and R₈ of a directly adjacent CR₅R₆ group or CR₇R₈ unit can stand also for a non-, mono- or disubstituted benzo ring or pyrido ring which is anellated on the X-C(R₅R₆) or X-C(R₇R₈) bond, the substituents of which can be selected from the group υ̂ and phenyl;
B and B' are selected independently of each other from one of the following groups a), b), c) or d), these being
a) mono-, di- and trisubstituted aryl radicals, the aryl radical being phenyl or naphthyl;
b) non-substituted, mono- and disubstituted heteroaryl radicals, the heteroaryl radical being pyridyl, furanyl, benzofuran-2-yl, benzofuran-3-yl, thienyl, benzothien-2-yl, benzothien-3-yl, or julolidinyl; the substituents of the aryl or heteroaryl radicals in a) and b) being those selected from the groups υ̂, β or δ or two directly adjacent substituents being able to form together a -D-(CH₂)ₖ-E-group which is bonded to the benzo ring, as defined above,
c) structural units with the following formulae (V) and (W): 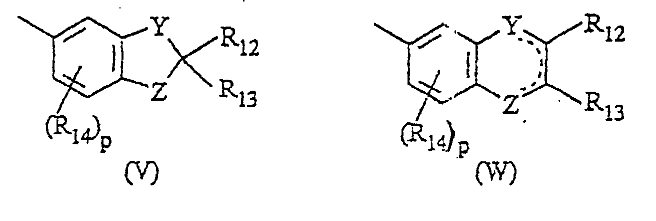 wherein
Y and Z are independently of each other O, S, CH, CH₂ or NR₁₁, the radical R₁₁ being defined as above, the radicals R₁₂ and R₁₃ representing independently of each other hydrogen and/or a (C₁-C₆)-alkyl radical and the radical R₁₄ being a substituent from the group υ̂, p being 1, 2 or 3, with the proviso that, if Y in the formula (V) is NR₁₁, Z is carbon
or
d) B and B' form together a non-, mono- or disubstituted fluoren-9-ylidene radical or a saturated hydrocarbon radical which is C₃-C₁₂ spiro-monocyclical, C₇-C₁₂ spiro-bicyclical and/or C₇-C₁₂ spiro-tricyclical, the fluorene substituents being selected from the group υ̂.

2. Photochromic benzo[f]chromenes according to claim 1, the cycle or heterocycle anellated in h-position of the benzo[f]chromene system according to the formula (I) being a five-membered, six-membered or seven-membered ring.

3. Photochromic benzo[f]chromenes according to claim 1 or 2, which have the following general formulae (II) or (III): 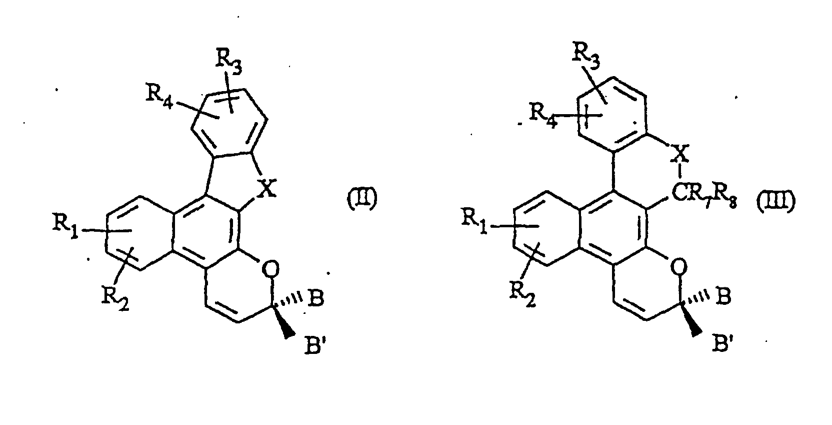 wherein B, B', R₁, R₂, R₃, R₄, R₇, R₈ and X are as defined above.

4. Photochromic benzo[f]chromenes according to one of the claims 1 to 3, the radicals R₁, R₂, R₃ and R₄ being selected from the groups υ̂, β and δ, with the proviso that, within the radicals R₁, R₂, R₃ and R₄, at least one is selected from the above group δ.

5. Photochromic benzo[f]chromenes according to claim 4, at least one of the radicals R₁ and R₂ being selected from the group δ.

6. Photochromic benzo[f]chromenes according to claim 5, the radical R₁ being selected from the group δ and being bonded in 7-position of the h-anellated 2H-benzo[f]chromene system.

7. Photochromic benzo[f]chromenes according to one of the claims 1 to 6, the substituent from the group δ being a non-, mono- or disubstituted amino group, the amine substituents being able to be selected from a (C₁-C₆)-alkyl radical, a (C₃-C₇)-cycloalkyl radical, a non-substituted phenyl radical or benzyl radical or a phenyl radical or benzyl radical substituted with one or more substituents from the group υ̂, an N-morpholine group, an N-piperidine group, an N-azacycloheptane group, an N-piperazine group, an N-(N'-(C₁-C₆-alkyl)piperazine group, an N-pyrrolidine group, an N-imidazolidine group or an N-pyrazolidine group.

8. Photochromic benzo[f]chromenes according to one of the claims 1 to 7, the radicals R₅, R₆, R₇ and R₈ respectively being selected independently of each other from the group α.

9. Photochromic benzo[f]chromenes according to one of the claims 1 to 8, X being O, CR₉R₁₀ or NR₁₁, the radicals R₉ R₁₀ and R₁₁ being defined as mentioned above.

10. Photochromic benzo[f]chromenes according to one of the preceding claims which are
7-dimethylamino-6-methoxy-2-(4-(N-morpholinyl)phenyl)-2-phenyl-2H,13H-indeno[1,2-h]benzo[f]chromene,
7-dimethylamino-6-methoxy-2-(4-(N-morpholinyl)phenyl)-2,13,13-triphenyl-2H,13H-indeno[1,2-h]benzo[f]chromene,
7-dimethylamino-6-methoxy-2-(4-(N-morpholinyl)phenyl)-2-phenyl-2H-13,14-dihydro-naphtho[1,2-h]benzo[f]chromene,
7-dimethylamino-6-methoxy-2-(4-(N-morpholinyl)phenyl)-2-phenyl-2H-benzofuro[3,2-h]benzo[f]chromene,
2-(4-dimethylaminophenyl)-6-methoxy-7-(N-piperidinyl)-2-phenyl-2H,13H-indeno[1,2-h]benzo[f]chromene,
2-(4-dimethylaminophenyl)-6-methoxy-7-(N-piperidinyl)-2,13,13-triphenyl-2H,13H-indeno[1,2-h]benzo[f]chromene,
2-(4-dimethylaminophenyl)-6-methoxy-7-(N-piperidinyl)-2-phenyl-2H-13,14-dihydro-naphtho[1,2-h]benzo[f]chromene,
2-(4-dimethylaminophenyl)-6-methoxy-7-(N-piperidinyl)-2-phenyl-2H-benzofuro[3,2-h]benzo[f]chromene,
2-(4-dimethylaminophenyl)-6-methoxy-7-(N-morpholinyl)-2-phenyl-2H,13H-indeno[1,2-h]benzo[f]chromene,
2-(4-dimethylaminophenyl)-6-methoxy-7-(N-morpholinyl)-2,13,13-triphenyl-2H,13H-indeno[1,2-h]benzo[f]chromene,
2-(4-dimethylaminophenyl)-6-methoxy-7-(N-morpholinyl)-2-phenyl-2H-13,14-dihydro-naphtho[1,2-h]benzo[f]chromene,
2-(4-dimethylaminophenyl)-6-methoxy-7-(N-morpholinyl)-2-phenyl-2H-benzofuro[3,2-h]benzo[f]chromene,
7-methoxy-6-(N-morpholinyl)-2-(4-(N-morpholinyl)phenyl)-2-phenyl-2H, 13H-indeno[1,2-h]benzo[f]chromene,
7-methoxy-6-(N-morpholinyl)-2-(4-(N-morpholinyl)phenyl)-2,13,13-triphenyl-2H,13H-indeno[1,2-h]benzo[f]chromene,
7-methoxy-6-(N-morpholinyl)-2-(4-(N-morpholinyl)phenyl)-2-phenyl-2H-13,14-dihydro-naphtho[1,2-h]benzo[f]chromene,
7-methoxy-6-(N-morpholinyl)-2-(4-(N-morpholinyl)phenyl)-2-phenyl-2H-benzofuro[3,2-h]benzo[f]chromene,
2-(4-dimethylaminophenyl)-9-methyl-2-phenyl-2H,9H,15H-7,8-dihydro-indeno[1,2-h]benz[1,4]oxazino[6,7-f]chromene,
2-(4-dimethylaminophenyl)-9-methyl-2,15,15-triphenyl-2H,9H,15H-7,8-dihydro-indeno[1,2-h]benz[1,4]oxazino[6,7-f]chromene,
2-(4-dimethylaminophenyl)-9-methyl-2-phenyl-2H,9H-7,8,15,16-tetrahydro-naphtho[1,2-h]benz[1,4]oxazino[6,7-f]chromene and
2-(4-dimethylaminophenyl)-9-methyl-2-phenyl-2H,9H-benzofuro[3,2-h]benz[1,4]-oxazino[6,7-f]chromene.

11. Use of the photochromic benzo[f]chromenes according to one of the claims 1 to 10 in plastic materials.

12. Use according to claim 11, the plastic material being an ophthalmic lens.

## Revendications

1. Benzo[f]chromènes photochromes annelés en h de formule générale (I) : 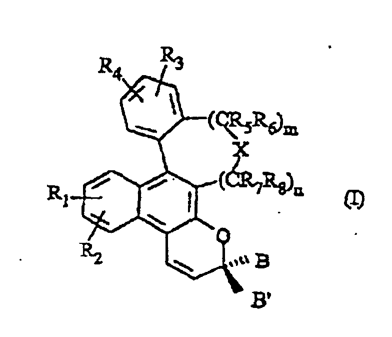 où
n et m indépendamment l'un de l'autre représentent 0, 1 ou 2,
les radicaux R₁, R₂, R₃ et R₄ représentent indépendamment l'une de l'autre un substituant, choisi parmi
le groupe α, constitué d'un atome d'hydrogène, d'un radical alkyle en (C₁-C₈), d'un radical cycloalkyle en (C₃-C₇) qui peut présenter un ou plusieurs hétéroatomes, d'un radical alkoxy en (C₁-C₆), d'un groupe hydroxy, d'un groupe trifluorométhyle, brome, chlore et fluor ;
le groupe β, constitué d'un radical phényle, phénoxy, benzyle, benzyloxy, naphtyle ou naphtoxy non substitué, mono ou disubstitué, les substituants pouvant être choisis parmi le groupe α et le phényle ;
le groupe χ, dans lequel les radicaux R₁ et R₂ ou R₃ et R₄ forment respectivement un groupe -A-(CH₂)ₖ-B- lié au cycle aromatique avec k=1 ou 2, A et B étant choisis indépendamment l'un de l'autre parmi l'oxygène, le soufre, CH₂, C(CH₃)₂ ou C(C₆H₅)₂ et un cycle benzène pouvant être annelé à nouveau à ce groupe -A-(CH₂)ₖ-B- ;
le groupe δ, constitué d'un groupe amino non substitué, mono ou disubstitué, les substituants amine pouvant être choisis parmi un radical alkyle en (C₁-C₆), un radical cycloalkyle en (C₃-C₇), un radical phényle ou benzyle non substitué ou substitué avec un ou plusieurs substituants issus du groupe α, d'un groupe N-morpholine, d'un groupe N-thiomorpholine, d'un groupe N-pipéridine, d'un groupe N-azacycloheptane, d'un groupe N-pipérazine, d'un groupe N-(N'-(C₁-C₆-alkyl)pipérazine, d'un groupe N-pyrrolidine, d'un groupe N-imidazolidine, d'un groupe N-pyrazolidine, d'un groupe N-aziridine, d'un groupe N-azétidine, d'un groupe N-indoline, d'un groupe N-carbazol, d'un groupe N-phénothiazine, d'un groupe N-phénazine, d'un groupe N-phénoxazine, d'un groupe N-tétrahydroquinoline et d'un groupe N-tétrahydroisoquinoline,
le groupe ε, dans lequel les radicaux R₁ et R₂ ou R₃ et R₄ forment respectivement un groupe -D-(CH₂)ₖ-E- lié au cycle aromatique avec k=1 ou 2, indépendamment l'un de l'autre, D étant choisi parmi N(CH₃) ou N(C₆H₅) et E parmi l'oxygène, le soufre, CH₂, C(CH₃)₂, C(C₆H₅)₂, N(CH₃) ou N(C₆H₅), et un cycle benzène pouvant être à nouveau annelé à ce groupe -D-(CH₂)ₖ-E-,
ou
le groupe φ, dans lequel les radicaux R₁ et R₂ ou R₃ et R₄ forment respectivement ensemble, avec le cycle benzène auquel ils sont liés, une unité julolidinyle,
avec la condition que, à l'intérieur des radicaux R₁, R_{2,} R₃ et R₄ ou à l'intérieur de R₁/R₂, et R₃/R₄ au moins l'un soit choisi parmi les groupes δ, ε et φ,
les radicaux R₅, R_{6,} R₇ et R₈ sont choisis respectivement indépendamment les uns des autres parmi le groupe α et le phényle ou les radicaux R₅ et R₆ forment ensemble avec le radical R₃ du cycle benzène directement adjacent un cycle benzène ou pyridine annelé à celui-ci, non substitué, mono- ou disubstitué, dont les substituants peuvent être choisis parmi le groupe α et le phényle, ou quand m ou n représentent 2, les radicaux directement adjacents R₅ et R₆ de deux unités CR₅R₆ ou les deux radicaux R₇ et R₈ de deux unités CR₇R₈ forment ensemble un cycle un cycle benzène ou pyridine annelé à celui-ci, non substitué, mono- ou disubstitué, dont les substituants peuvent être choisis parmi le groupe α et le phényle, ou les radicaux R₅ et R₆ et/ou les radicaux R₇ et R₈ représentent ensemble un radical (C₃-C₇)cycloalkyle qui peut présenter un ou plusieurs hétéroatomes, un cycle benzène pouvant être annelé à ce radical cycloalkyle ;
X est choisi parmi O, S, CR₉R₁₀ ou NR₁₁, le radical R₁₁ pouvant être hydrogène, cyano, alkyle en (C₁-C₆), cycloalkyle en (C₃-C₇) ou phényle, et les radicaux R₉ et R₁₀, indépendamment l'un de l'autre, étant choisis parmi le groupe α et phényle ou les radicaux R₉ et R₁₀, ensemble, représentent un radical cycloalkyle en (C₃-C₇) qui peut présenter un ou plusieurs hétéroatomes ou quand X est CR₉R₁₀, les radicaux R₉ et R₁₀, ensemble, avec les radicaux R₅ et R₆, ou R₇ et R₈ d'un groupe CR₅R₆ ou une unité CR₇R₈ directement adjacent pouvant représenter également un cycle benzène ou pyridine non substitué, monosubstitué ou disubstitué annelé à une liaison X-C(R₅R₆) ou X-C(R₇R₈), dont les substituants peuvent être choisis parmi le groupe α et le groupe phényle ;
B et B', indépendamment l'un de l'autre, est choisi parmi l'un des groupes suivants a), b), c) ou d), B et B' étant
a) des radicaux aryle mono-, di- et trisubstitués, le radical aryle étant le phényle ou le naphtyle ;
b) des radicaux hétéroaryle non substitués, mono- et disubstitués, le radical hétéroaryle étant le pyridyle, le furanyle, le benzofuran-2-yle, le benzofuran-3-yle, le thiènyle, le benzothièn-2-yle, le benzothièn-3-yle ou le julolidinyle ;
les substituants des radicaux aryle ou hétéroaryle au a) et b) étant ceux choisis parmi les groupes α, β ou δ ou deux substituants directement adjacents pouvant former un groupe -D-(CH₂)ₖ-E-, comme défini précédemment,
c) les unités structurales avec les formules suivantes (V) et (W) sont : 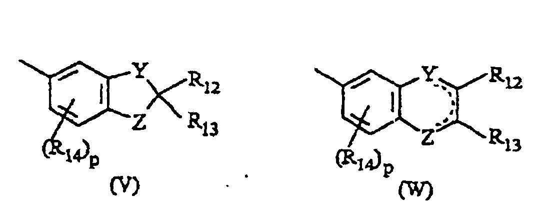 où
Y et Z sont indépendamment l'un de l'autre O, S, CH, CH₂ ou NR₁₁, le radical R₁₁ est tel que défini précédemment, les radicaux R₁₂ et R₁₃ représentant indépendamment l'un de l'autre l'hydrogène et/ou un radical alkyle (en C₁-C₆) et le radical R₁₄ est un substituant du groupe α, p étant 1, 2 ou 3, avec la condition que quand Y dans la formule (V) est NR11, Z est carbone,
d) B et B' ensemble, forment un radical fluorène-9-ylidène ou un radical saturé hydrate de carbone, qui est spiro-monocyclique en C₃-C₁₂, spiro-bicylique en C₇-C₁₂ et/ou spiro-tricyclique en C₇-C₁₂, les substituants fluorène étant choisis parmi le groupe α.

2. Benzo[f]chromènes photochromes selon la revendication 1, où le cycle ou hétérocycle annelé en position h du système benzo[f]chromène selon la formule (I) est un pentacycle, hexacycle ou heptacycle.

3. Benzo[f]chromènes photochromes selon la revendication 1 ou 2, présentant les formules générales (II) ou (III) : 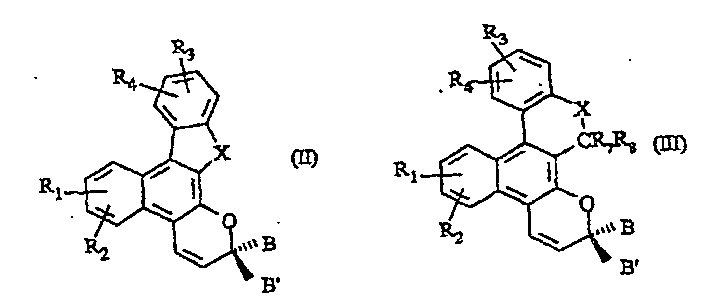 dans lesquelles B, B', R₁, R₂, R₃, R₄, R₇, R₈ et X sont tels que définis précédemment.

4. Benzo[f]chromènes photochromes selon l'une des revendications 1 à 3, où les radicaux R₁, R₂, R₃ et R₄ sont choisis parmi les groupes α, β et δ, avec la condition que, à l'intérieur des radicaux R₁, R₂, R₃ et R₄, au moins l'un soit choisi parmi le groupe précédent δ.

5. Benzo[f]chromènes photochromes selon la revendication 4, où au moins l'un des radicaux R₁ et R₂ est choisi parmi le groupe δ.

6. Benzo[f]chromènes photochromes selon la revendication 5, où le radical R₁ est choisi parmi le groupe δ et est lié en position 7 du système 2H-benzo[f]chromène annelé en h.

7. Benzo[f]chromènes photochromes selon l'une des revendications 1 à 6, où le substituant du groupe δ est un groupe amino non substitué, mono ou disubstitué, les substituants amine pouvant être choisis parmi un radical alkyle en (C₁-C₆), un radical cycloalkyle en (C₃-C₇), un radical phényle ou benzyle non substitué ou substitué avec un ou plusieurs substituants issus du groupe α, d'un groupe N-morpholine, d'un groupe N-pipéridine, d'un groupe N-azacycloheptane, d'un groupe N-pipérazine, d'un groupe N-(N'-(C₁-C₈-alkyl)pipérazine, d'un groupe N-pyrrolidine, d'un groupe N-imidazolidine ou d'un groupe N-pyrazolidine.

8. Benzo[f]chromènes photochromes selon l'une des revendications 1 à 7, où les radicaux R₅, R₆, R₇ et R₈ sont choisis indépendamment les uns des autres parmi le groupe α.

9. Benzo[f]chromènes photochromes selon l'une des revendications 1 à 8, X est O, CR₉R₁₀ ou NR₁₁, les radicaux R₉, R₁₀ et R₁₁ étant tels que définis précédemment.

10. Benzo[f]chromènes photochromes selon l'une quelconque des revendications précédentes, qui sont :
7-diméthylamino-6-méthoxy-2-(4-(N-morpholinyl)phényl)-2-phényl-2H,13H-indèno[1,2-h]benzo[f]chromène,
7-diméthylamino-6-méthoxy-2-(4-(N-morpholinyl)phényl)-2-phényl-2,13,13-triphényl-2H,13H-indèno[1,2-h]benzo[f]chromène,
7-diméthylamino-6-méthoxy-2-(4-(N-morpholinyl)phényl)-2-phényl-2H-13,14-dihydro-naphto[1,2-h]benzo[f]chromène,
7-diméthylamino-6-méthoxy-2-(4-(N-morpholinyl)phényl)-2-phényl-2H-benzofuro[3,2-h]benzo[f]chromène,
2-(4-diméthylaminophényl)-6-méthoxy-7-(N-pipéridinyl)-2-phényl-2H,13H-indèno[1,2-h]benzo[f]chromène,
2-(4-diméthylaminophényl)-6-méthoxy-7-(N-pipéridinyl)-2,13,13-triphényl-2H,13H-indèno[1,2-h]benzo[f]chromène,
2-(4-diméthylaminophényl)-6-méthoxy-7-(N-pipéridinyl)-2-phényl-2H-13H,14-dihydro-naphtho[1,2-h]benzo[f]chromène,
2-(4-diméthylaminophényl)-6-méthoxy-7-(N-pipéridinyl)-2-phényl-2H-benzofuro[1,2-h]benzo[f]chromène,
2-(4-diméthylaminophényl)-6-méthoxy-7-(N-morpholinyl)-2-phényl-2H,13H-indèno[1,2-h]benzo[f]chromène,
2-(4-diméthylaminophényl)-6-méthoxy-7-(N-morpholinyl)-2,13,13-triphényl-2H,13H-indèno[1,2-h]benzo[f]chromène,
2-(4-diméthylaminophényl)-6-méthoxy-7-(N-morpholinyl)-2-phényl-2H-13,14-dihydro-naphto[1,2-h]benzo[f]chromène,
2-(4-diméthylaminophényl)-6-méthoxy-7-(N-morpholinyl)-2-phényl-2H-benzofuro[3,2-h]benzo[f]chromène,
7-méthoxy-6-(N-morpholinyl)-2-(4-(N-morpholinyl)phényl)-2-phényl-2H-13H-indèno[1,2-h]benzo[f]chromène,
7-méthoxy-6-(N-morpholinyl)-2-(4-(N-morpholinyl)phényl)-2,13,13-triphényl-2H-13H-indèno[1,2-h]benzo[f]chromène,
7-méthoxy-6-(N-morpholinyl)-2-(4-(N-morpholinyl)phényl)-2-phényl-2H-13,14-dihydro-naphto[1,2-h]benzo[f]chromène,
7-méthoxy-6-(N-morpholinyl)-2-(4-(N-morpholinyl)phényl)-2-phényl-2H-benzofuro[3,2-h]benzo[f]chromène,
2-(4-diméthylaminophényl)-9-méthyl-2-phényl-2H,9H,15H-7,8-dihydro-indèno[1,2-h]benz[1,4]oxazino[6,7-f]chromène,
2-(4-diméthylaminophényl)-9-méthyl-2,15,15-triphényl-2H,9H,15H-7,8-dihydro-indèno[1,2-h]benz[1,4]oxazino[6,7-f]chromène,
2-(4-diméthylaminophényl)-9-méthyl-2-phényl-2H,9H-7,8,15,16-tétrahydro-naphto[1,2-h]benz[1,4]oxazino[6,7-f]chromène et
2-(4-diméthylaminophényl)-9-méthyl-2-phényl-2H,9H-benzofuro[3,2-h]benz[1,4]oxazino[6,7-f]chromène.

11. Utilisation des benzo[f]chromènes photochromes selon l'une des revendications 1 à 10 dans les matières plastiques.

12. Utilisation selon la revendication 11, la matière plastique étant une lentille ophtalmique.
